# EUROPÄISCHE PATENTSCHRIFT

(11) **EP 0 792 145 B1**
(45) Veröffentlichungstag und Bekanntmachung des Hinweises auf die Patenterteilung: **28.10.1998**
(21) Anmeldenummer: 95942687.5
(22) Anmeldetag: 18.12.1995
(51) Int. Cl.: A61K 9/70, A61K 47/12, A61K 47/10

(54) **TRANSDERMALE RESORPTION VON WIRKSTOFFEN AUS UNTERKÜHLTEN SCHMELZEN**
TRANSDERMAL RESORPTION OF ACTIVE SUBSTANCES FROM SUPERCOOLED MASSES
RESORPTION TRANSDERMIQUE DE PRINCIPES ACTIFS CONTENUS DANS DES MASSES SURFONDUES

(30) Priorität: 24.12.1994 DE 4446600
(43) Veröffentlichungstag der Anmeldung: 03.09.1997
(73) Patentinhaber: LTS LOHMANN Therapie-Systeme GmbH, 56567 Neuwied (DE)
(72) Erfinder: HILLE, Thomas, 54545 Neuwied (DE)
(74) Vertreter: Flaccus, Rolf-Dieter, Dr.
(86) Internationale Anmeldenummer: EP9505006
(87) Internationale Veröffentlichungsnummer: WO9619975

(56) Entgegenhaltungen:
- EP-A- 0 430 019
- WO-A-91/04733
- WO-A-91/19474
- DATABASE WPI Week 8335 Derwent Publications Ltd., London, GB; AN 83-750625 XP002002272 & JP,A,58 124 711 (KANEBO KK) , 25.Juli 1983

## Beschreibung

Die Erfindung betrifft die transdermale Applikation von Arzneistoffen unter Zuhilfenahme von Hilfstoffen, die unterkühlte Schmelzen bilden. Den zweifellos großen Vorteilen, die die transdermale Applikation von Pharmaka aufweist, steht oft als Nachteil die Limitierung der Menge des Arzneistoffs gegenüber, die über die Haut aufgenommen werden kann. Daher wurde gleichzeitig mit Beginn der Therapie durch transdermale Applikation nach Wegen gesucht, die Penetrationsfähigkeit von Pharmaka durch die Haut zu erhöhen. Ein Lösungsansatz wird in der Entwicklung von Penetrationsförderern gesehen, die Arzneimitteln zur dermalen Applikation zugefügt werden. Diese Substanzen verändern zumindest für einen kurzen Zeitraum tiefer liegende Hautstrukturen und können in ungünstigen Fallen zu unerwünschten Nebeneffekten führen. Diese Nebeneffekte sind umso weniger ausgeprägt, je besser die Penetrationsförderer physiologisch verträglich sind.
Ein eleganter Lösungsansatz, um die Resorption von Arzneistoffen durch die Haut zu steigern, ist das Herstellen von Matrixsystemen. Aufgrund des Konzentrationsgefälles diffundiert zunächst der gelöste Anteil des Wirkstoffs aus dem System in die Haut hinein. Gleichzeitig wird der Teil des Wirkstoffs, der suspendiert im System enthalten ist, nachgelöst. Die Auflösungsgeschwindigkeit des Wirkstoffs im System ist der geschwindigkeitsbestimmende Schritt bei der Arzneistoffabgabe. Experimentell konnte gezeigt werden, daß Dodecanol penetrationsfördernd wirkt und physiologisch unbedenklich ist. Da sein Festpunkt bei 24°C liegt, ist Dodecanol bei üblicher Raumtemperatur fest. Die Bereitstellung von Dodecanol aus einer Matrix ist durch den festen Aggregatzustand erschwert. Dieses Manko schränkt die Einsatzmöglichkeiten von Dodecanol in Polymermatrices ein, denn die Abgaberate eines Stoffes aus Polymermatrices ist umso größer, je leichter er in der Matrix diffunderieren kann. Dies gilt nicht nur für Dodecanol, sondern auch für andere Penetrationsförderer, die bei Raumtemperatur fest sind.
Aufgabe der Erfindung ist daher die Verbesserung der Abgabe von Hilfsstoffen, die bei Raumtemperatur fest sind, aus einer Matrix.

Diese Aufgabe wird erfindungsgemäß überraschenderweise gelöst durch ein Arzneimittel nach Anspruch 1. Unter Verbindungen, die unterkühlte Schmelzern bilden, werden solche verstanden, deren Schmelzpunkt über Raumtemperatur liegt, die aber nach einem Aufschmelzvorgang beim Abkühlen auf Raumtemperatur im flüssigen Zustand verharren.

Dem Fachmann ist der Begriff Arzneimittel bekannt. Zur Applikation auf der Haut oder auf Schleimhäuten (z.B. Nase, Auge) sind Salben, die Gele von plastischer Verformbarkeit darstellen, geeignet, ebenso Pasten, die als Salben mit hohem Feststoffanteil bezeichnet werden können.

Unter einem transdermalen therapeutschen System (TTS) soll nach Zafaroni "eine arzneistoffenthaltende Vorrichtung bzw. eine Darreichungsform, die einen Arzneistoff oder mehrere in vorausbestimmter Rate kontinuierlich über einen festgesetzten Zeitraum an einen festgelegten Anwendungsort abgibt" (zitiert nach Heilmann, therapeutische Systeme - Konzept und Realisation programmierter Arzneiverabreichung, 4. Auflage, Ferdinand Enke-Verlag Stuttgart 1984, Seite 26) verstanden werden, wobei im vorliegenden Fall der Anwendungsort die Haut ist.

Der Aufbau von transdermalen Systemen ist dem Fachmann bekannt. Schutzrechte, in denen der grundsätzliche Aufbau beschrieben wird, sind beispielsweise DE 3315272, DE 3843239, US 3,598,122.

Wird ein transdermales therapeutisches System auf die Haut eines Patienten appliziert, soll der Arzneistoffabgegeben werden, um beim Patienten topisch oder systemisch wirksam zu werden. Arzneiformen dieser Art werden bereits therapeutisch genutzt. Sie sind meist schichtförmig aufgebaut und bestehen im einfachsten Falle aus einer Rückschicht, einem selbstklebenden Wirkstoffreservoir und einer wieder ablösbaren Schutzschicht, die vor der Applikation zu entfernen ist. Im vorliegenden Fall muß ein Hilfsstoff eingeführt werden, der die Resorption von Arzneistoffen durch die Haut steigert, bei Raumtemperatur fest ist und unterkühlte Schmelzen bildet, z.B. Dodecanol oder Lävulinsäure.

Als Arzneistoffe werden Substanzen verwendet, die ohne oder mit Resorptionsfilter auf der Haut appliziert, eine lokale oder systemische Wirkung hervorrufen.

Stoffe mit lokaler Wirkung sind z.B. Antitransperantia, Fungizide, Bakterizide und Bakteriostatika.

Stoffe mit systemischer Wirkung sind beispielsweise Antibiotika, Hormone, Antipyretika, Antidiabetika, Koronardilatatoren, herzwirksame Glycoside, Analgetika, Spasmolytika, Antihypertonika, Psychopharmaka, Migränemittel, Kortikoide, Kontrazeptiva, Antirheumatika, Anticholinergika, Symphatikolytika, Symphatikomimetika, Vasodilatatoren, Antikoagulantien und Antirhythmica. Diese Aufzählung ist nicht vollständig.

Die Abgabe von Pilocarpinbase oder Ephedrinbase aus Polymermatrices läßt sich ebenfalls steigern, wenn diese in unterkühlter Schmelzen vorliegen. Ein Aufschmelzen dieser Arzneistoffe verbietet sich jedoch, da sie temperaturlabil sind.

Die Erfindung wird durch folgende Beispiele erläutert:
- **Beispiel 1 :**: 1.139 g einer 47,83 Gew.%-igen Polyacrylatlösung eines selbstvernetzten Acrylatcopolymeren aus 2-Ethylhexylacrylat, Vinylacetat, Acrylsäure (Lösemittel : Ethylacetat : Heptan : Isopropanol : Toluol : Acetylacetonat im Verhältnis von 37 : 26 : 26 : 4 : 1), 100 g Lävulinsäure, 150 g Oleyloleat, 100 g Polyvinylpyrolidon, 150 g Ethanol, 200 g Ethylacetat und 100 g Buprenorphinbase werden homogenisiert. Man rührt etwa 2 Stunden und kontrolliert visuell, ob alle Feststoffe gelöst sind. Man kontrolliert den Verdunstungsverlust durch Zurückwiegen und ergänzt ggfls. den Lösemittelverlust durch Ethylacetat.
Anschließend wird die Mischung so auf eine 420 mm breite transparente Polyesterfolie aufgetragen, daß das Flächengewicht der getrockneten Kleberschicht bei 80 g pro m² liegt. Die durch Silikonbehandlung wieder ablösbare Polyesterfolie dient als Schutzschicht.
Man entfernt die Lösemittel durch Trocknen mit erwärmter Luft, die über die feuchte Bahn geleitet wird. Durch die Wärmebehandlung verdampfen nicht nur die Lösemittel, sondern es schmilzt auch die Lävulinsäure. Anschließend deckt man den Kleberfilm mit einer Polyesterfolie 15 µ ab. Mit geeigneten Schneidewerkzeugen stanzt man eine Fläche von 16 cm² aus und entfernt die Ränder, die zwischen den einzelnen Systemen stehengeblieben sind. Die Beispiele 2 bis 5 wurden analog Beispiel 1 ausgeführt.
Spalte 1 gibt die Versuchsnummer an, Spalte 2 die Konzentration von Buprenorphinbase, Spalte 3 die qualitative und quantitative Angabe der eingesetzten Säure, Spalte 4 die Dissoziationskonstanten der eingesetzten Säuren, Spalte 5 den Weichmacher, Spalte 6 die eingesetzten Polymere und Spalte 7 die Resorptionsrate unter in vitro Bedingungen an. Angegeben ist die relative Menge Buprenorphinbase, die durch Mäusehaut in 24 h diffundiert, bezogen auf die Menge pro TTS. Die Penetrationsergebnisse wurden mit Mäusehaut gewonnen, die in eine Franzzelle gespannt wurde. Die Zusammensetzung der Beispiele 2 - 5 sind in Tab. 1 wiedergegeben.

Die Ergebnisse der in-vitro-Penetrationsversuche sowie die qualitativen und quantitativen Zusammensetzungen von Buprenorphin-TTS gemäß Beispiel 1 - 5 sind in Tab. 1 dargestellt :

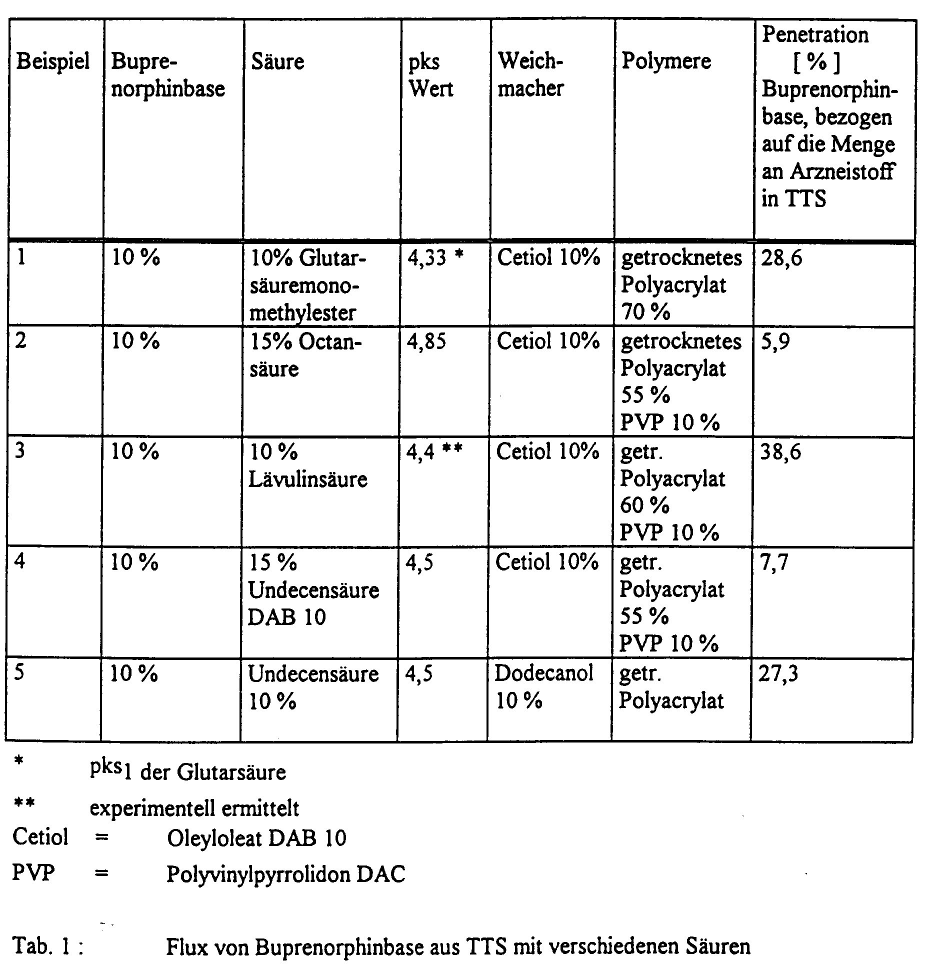

Aus Spalte 4 erkennt man, daß die Dissoziationskonstanten der eingesetzten Karbonsäuren durchaus ähnlich sind. Setzt man in jedem Fall den Weichmacher Oleyloleat in 10%-iger Konzentration ein, so erkennt man jedoch aus den Beispielen 1 und 3 bzw. 2 und 4, daß nur die Karbonsäuren, die zum Ausbilden von unterkühlten Schmelzen neigen, eine deutliche Penetrationssteigerung unter in vitro Bedinungen bewirken. Undecensäure und Octansäure sind, wie man aus den Dissovationskonstanten erkennen kann, schwächere Säuren als Glutarsäuremonomethylester oder Lävulinsäure. Dies wurde dadurch ausgeglichen, daß die Konzentration der beiden schwächeren Säuren mit 15 % höher liegt als die Konzentration der beiden stärkeren Säuren. Gleichzeitig ist jedoch erkennbar, daß die Dissovationskonstanten so nahe beieinander liegen, daß der Effekt der Resorptionsförderung nicht durch die Dissozationskonstanten erklärt werden kann. Beispiel 5 seigt, daß auch mit einer Säure, die offensichtlich nur wenig Effekt auf die Penetrationsförderung von Buprenorphinbase hat, eine Resorptionssteigerung erreicht werden kann, wenn ein Neutralstoff, nämlich Dodecanol, eingesetzt wird, der wie Lävulinsäure oder Glutarsäuremonomethylester unterkühlte Schmelzen bildet. Durch die 5 Beispiele ist der physikalische Effekt der angegebenen Hilfs-stoffe hinreichend belegt.

Eine Rekristallisation der Lävulinsäure bzw. des Dodecanols bei Lagerung trat bei keinem der Muster auf, auch dann nicht, wenn bei 4°C gelagert wurde.

## Patentansprüche

1. Arzneimittel zur Abgabe von Arzneistoffen an die Haut mit resorptionsteigernden Hilfsstoffen, dadurch gekennzeichnet, daß der resorptionsteigernd wirkende Hilfsstoff Lävulinsäure in unterkühlter Schmelze ist.

2. Arzneimittel nach Anspruch 1, dadurch gekennzeichnet, daß der Arzneistoff Buprenorphinbase und der resorptionssteigernd wirkende Hilfsstoff Lävulinsäure in unterkühlter Schmelze ist.

## Claims

1. A drug for the release of medicinal agents to the skin comprising auxiliary agents having absorption-increasing effect, characterized in that the auxiliary agent having absorption-enhancing effect is levulic acid in subcooled melt.

2. A drug according to claim 1 characterized in that the medicinal agent is buprenorphine base and the auxiliary agent having absorption-increasing effect is levulic acid in subcooled melt.

## Revendications

1. Médicament pour la libération de principes actifs à la peau au moyen d'adjuvants augmentant la résorption, caractérisé en ce que l'adjuvant à action d'augmentation de la résorption est l'acide lévulinique en surrefroidissement.

2. Médicament selon la revendication 1, caractérisé en ce que le principe actif est la base de buprénorphine et l'adjuvant à action d'augmentation de la résorption est l'acide lévulinique en surrefroidissement.
